(19) **Europäisches Patentamt**

**European Patent Office**

**Office européen des brevets**

(11) **EP 0 634 184 B1**

(12) **EUROPEAN PATENT SPECIFICATION**

(45) Date of publication and mention
of the grant of the patent:
**24.10.2001   Bulletin 2001/43**

(51) Int Cl.⁷: **A61M 15/00**

(21) Application number: **94305150.8**

(22) Date of filing: **14.07.1994**

(54) **Dry powder inhalers**

Trockenpulverinhalator

Inhalateur à poudre sèche

(84) Designated Contracting States:
**CH DE ES FR GB IT LI SE**

(30) Priority:   **14.07.1993   GB 9314614**

(43) Date of publication of application:
**18.01.1995   Bulletin 1995/03**

(73) Proprietor: **AstraZeneca AB**
**151 85 Södertälje (SE)**

(72) Inventors:
 • **Smith, David K.**
   **Loughborough LE 11 1EP Leicestershire (GB)**
 • **Hodson, Peter D.**
   **Loughborough LE 11 1EP Leicestershire (GB)**
 • **Wass, Anthony C.L.**
   **Loughborough LE 11 1 EP, Leicestershire (GB)**

(74) Representative: **Bowman, Paul Alan et al**
   **LLOYD WISE, TREGEAR & CO.,**
   **Commonwealth House,**
   **1-19 New Oxford Street**
   **London WC1A 1LW (GB)**

(56) References cited:
   **WO-A-90/13328          WO-A-93/09832**

Remarks:
The file contains technical information submitted
after the application was filed and not included in this
specification

## Description

This invention relates to dry powder inhalers and in particular to dry powder inhalers in which powdered medicament is carried on the surface of a flexible sheet material prior to dispensing.

Asthma and other respiratory diseases have long been treated by the inhalation of appropriate medicament. For many years the two most widely used and convenient choices of treatment have been inhalation of medicament from a drug solution or suspension in a metered dose inhaler (MDI), or inhalation of powdered drug and perhaps excipients from a dry powder inhaler (DPI). With growing concern being voiced over the damage done to the earth's ozone layer by CFC's (chlorofluorocarbons) which are in widespread use in existing MDI'S, heightened interest in DPI systems has been stimulated.

W090/13328 discloses a dry powder inhalation device comprising a housing defining a chamber in communication with a patient port in the form of a mouthpiece or nasal adaptor, and an elongate carrier bearing a powdered medicament, the device being constructed and arranged such that areas of predetermined size of the elongate carrier may sequentially be exposed within the chamber, the device comprising one or more air inlets such that when a patient inhales through the patient port an air flow is established from the air inlet(s) to the patient port through the chamber such that particles of the powdered medicament of respirable size from said exposed area of the elongate carrier are entrained within the air flow.

The device generally comprises means to advance the elongate carrier to expose an area of said carrier within the chamber, said means being operable prior to or during patient inhalation through the patient port and means for releasing medicament of respirable size from the exposed area of carrier, e.g. in the form of means for impacting or striking the exposed area of the carrier either singularly or by a plurality of such strikings or impactions.

W092/08509 discloses a breath-actuated triggering mechanism which is suitable for use in such a device and WO-A-9 309 832 claiming the priority of GB Patent Application No. 9123953 discloses a deagglomerator suitable for use with dry powder inhalers.

EP-455463 discloses the use of microdimpled materials to hold doses of powdered medicament for inhalation therapy. Such materials may be in the form of an elongate substrate in which a surface of the substrate comprises:

(i) one or more grooves of width 10 to 500$\mu$m at the carrier surface and depth 10 to 500$\mu$m, the grooves containing particles of powdered medicament,
(ii) randomly orientated pores of diameter 0.1 to 100$\mu$m, at least a portion of the pores being on the exterior surface and containing particles of powdered medicament,
(iii) apertures of diameter 1 to 100$\mu$ m in at least one surface produced by laser drilling, the apertures containing particles of powdered medicament, or,
(iv) an embossed surface.

It has been found that the particular form of an impactor for dislodging powdered medicament from a carrier can significantly affect the amount of medicament dislodged and hence the performance level of the dry powder inhaler.

According to the present invention there is provided a device for delivering powder comprising a flexible sheet material (27, 122) carrying a powder to be delivered on a surface thereof, structure to support a substantially planar portion of the sheet material and a resettable impactor (32, 128) for dislodging said powder from said portion of the sheet material, the impactor comprising a curved head which is movable between a first, primed position distant from said planar portion of the sheet material, and a second position in which the head impacts a substantially planar portion of said flexible sheet material, the impactor being resiliently biased to propel the head from said first towards said second position, characterised in that the impactor (32, 128) comprises:

(a) a shaft (6) having a first end (2, 130) that is mounted within the device, and
(b) a cantilevered arm (8, 138) extending from said shaft terminating in a free end, said cantilevered arm comprising the impactor head

wherein the movement of said shaft between said first and second positions defines a plane of motion,
at least a portion of said cantilevered arm, disposed intermediate (i) the intersection of said arm with said shaft and (ii) said free end, extending out of a first plane that includes said shaft and is perpendicular to said plane of motion, towards a second plane that is defined by said substantially planar portion of said flexible sheet material,
a second portion of said cantilevered arm (8, 138) between said intermediate portion and said free end intersects said first plane, defining a curve,
and said free end extends out of said first plane in a direction away from said second plane.

The impactor of the present invention has a form designed to provide uniform impactions to provide consistent and effective release of powder from the sheet material. The form of the impactor is also designed to provide uniform resetting and triggering release actions. It is also designed to be as independent as possible of manufacturing tolerances, e.g. by appropriate selection of the shape of tail, by having at least two turns in the spring region, by curvature of the head, etc.

The curved shape of the head, in particular, confers several advantages, referred to below. Experimental results have shown that the need for such a curved, springy head is crucial if shock waves in the tape to produce adequate powder release are to be obtained. Use of pressed steel impactor heads on plastic impactor bodies, or even of short metal spring wire heads rigidly attached at the elbow to a rigid impactor body, both gave rise to much reduced drug release, demonstrating the need for a springy, undamped impactor arm and head.

The impactor is preferably in the form of a single component and comprises an integral spring to store energy for impaction, together with a curved head region for striking the powder bearing tape. The nature of the impactor is important, as it must be sufficiently springy to be able to elastically store energy and to transmit that energy efficiently via the head region to the sheet material. A preferred form of the impactor is bent from a length of steel spring wire. The curved shape of the head region is particularly critical.

The impactor of the invention is useful in dry powder inhalers, particularly inhalers disclosed in W090/13328, W092/08509 and GB9123953.

The invention will now be described with reference to the accompanying drawings in which:

Figures 1(a) to (c) represent plan, side and end views of an impactor in accordance with the invention,
Figure 2 represents a diagram of a breath-actuated dry powder inhaler in accordance with the invention, and
Figure 3 represents a diagram of a manually operated dry powder inhaler.

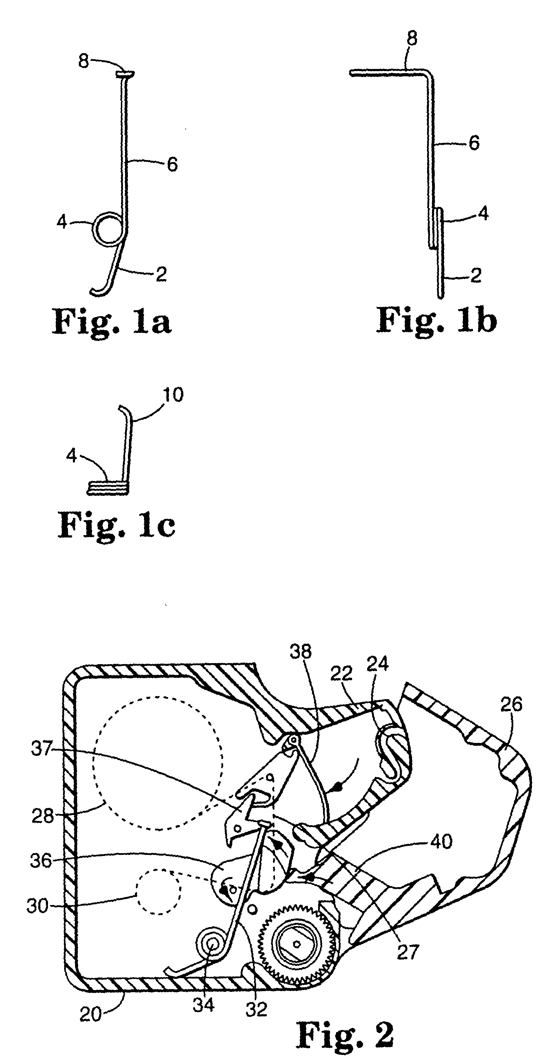

The impactor shown in Figure 1 is a single component formed from spring steel. It has four main regions, the tail (2), the spring (4), the shaft (6) and the head in the form of a cantilevered arm (8). For an exemplary total length of ~96mm, the different regions may comprise ~14, 38, 29, 15mm respectively.

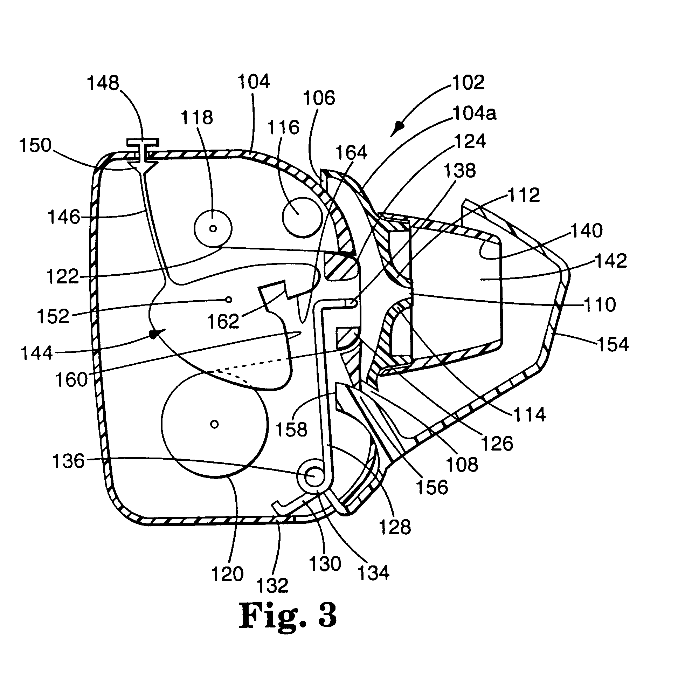

The function of the tail region (2) is to provide anchorage of the end of the spring region (4) against rotation during resetting of the impaction mechanism (storage of energy in the impactor by torsion of the coils of the spring). As such the tail (2) is designed to bear on the outer wall of the device body during resetting (Figures 2 and 3). The tail (2) or other fixed portion may be curved to prevent it from damaging this plastic wall or from "digging in" and thus altering the setting point, etc.

The function of the spring region (4) is to allow sufficient storage of energy in the impactor. To do this generally requires multiple coils to allow enough elastic potential energy to be stored without leading to undue strains on any length of the wire which might lead to plastic deformation, fatigue fracturing, etc. More turns, however, give rise to a deeper spring and hence lead to a greater space requirement in the device. For many devices, two turns are adequate. The turns must be designed not to tighten on the locating peg (Figures 2 and 3).

The shaft region (6) of the impactor serves several functions. It allows a greater length of impactor head travel than resetting arm motion and allows the impactor head motion to be particularly springy, i.e. it can create a whiplash effect as stored potential energy is released during travel, and it serves as the part of the impactor against which the activator component pushes during resetting (Figure 2) and the part which pushes the activator component out of the way during impactor flight.

The impactor head which is in the form of a cantilevered arm (8) is the most critical region of all. The crown (10) of the head (8) is curved, e.g. 10mm radius and it is aligned to strike the centerline of the medicament tape to ensure central impaction. This curvature also serves to give consistent impactions, no matter whether the tape lies at a slight angle to that intended. If the head were straight, then any small misalignments of the tape prior to an impaction would tend to give inconsistent behavior, with the impactor head sometimes striking parallel along its length, sometimes striking the tape with its top corner first, or sometimes striking the tape first with its elbow where it joins the arm region. In addition, the curvature of the head allows the tape to be bent into a three-dimensional surface as it is struck, giving rise to more complex vibrations in the tape and thus serving better to remove and aerosolise powder from it. Because only the center of the tape is initially contacted by the impactor head, the vibrations set up are less likely to be damped out by contact with the head. Furthermore, the curvature ensures that the end of the impactor wire cannot reach the tape and damage, perforate, or even break it. Finally, the curved top of the impactor steers the tape correctly to the front of the impactor head during insertion of a new cassette of tape by the patient, thereby ensuring that the tape cannot be inserted behind the impactor.

The end of the cantilevered arm (8) may be bent further, through 180°, to reduce the risk of a patient scratching their finger on it. Alternatively, the end may be dipped, to form a small spherical head for the same reason. Preferably, a tunnel should cover the impactor head to prevent the patient being able to contact the impactor in inhalation devices.

The cantilevered arm (8) should be as low in mass as possible, commensurate with having sufficient strength and rigidity to strike the tape in the correct position and at the correct time. The reason for a low mass requirement is that this characteristic allows maximum transfer of energy to the medicament coated tape. Considering the impactor head as an object of mass $M_H$ and initial velocity $V_H$ ($V_{H2}$ after collision), and a drug particle as an initially stationary object of mass $M_P$ and final velocity $V_P$ after being struck by the head:

$$M_H V_H = M_H V_{H2} + M_P V_P$$

and

$$M_H V_H{}^2 = M_H V_{H2}{}^2 + M_P V_P{}^2$$

in a two body system assuming no losses and where momentum and kinetic and kinetic energy are conserved.

If $M_H = k M_P$ then substitution leads to:

$$V_P/V_H = \frac{2\,k}{k+1}$$

From this equation come the following observations:

As $k \to 0$, $V_P/V_H \to 0$

i.e. a small impactor does not move a large drug particle.

For $k = l$, $V_P/V_H = 1$

i.e. Newton's cradle situation

As $k \to \infty$, $V_P/V_H \to 2$

i.e. the maximum possible velocity of the drug particle is twice that of the incoming impactor (this can be viewed as the drug particle approaching at $-V_H$ and bouncing off at $+V_H$ in the impactor's frame of reference). Hence in order to impart as much velocity as possible to the drug particles (i.e. in order to transfer as much kinetic energy as possible to them), the velocity of the impactor should be as high as possible, i.e. the impactor head should have as low a mass as possible for a given kinetic energy stored in the spring region of the impactor.

For an impactor shown in Figure 1, typical times of flight of approximately lms are needed to travel ∼10mm to the tape, i.e. mean speed ∼10m/s; maximum speed just prior to impaction of the rear of the powder coated tape being in excess of this.

The impactor is stopped just after striking the tape, because follow-through could cause tearing of the tape, could pull a loop of excess tape off the supply spool, cause the tape to block the airflow channel, etc.

Figure 2 illustrates a dry powder inhaler of the type disclosed in W090/13328, W092/08509 and GB 9123953; the full details of operation of the inhaler are disclosed in these references, the full disclosures of which are incorporated herein by reference. Figure 2 shows the inhaler during closure of the mouthpiece cover.

The inhaler comprises a housing (20), mouthpiece (22) incorporating a deagglomerator (24) and a mouthpiece cover (26). The dry powder medicament is carried on an elongate carrier shown in dashed outline (27) which extends from a supply spool (28) to a take-up spool (30).

The inhaler comprises an impactor (32) which may have the form as shown in Figure 1 and is positioned with the coil of the spring over locating peg (34).

The impactor (32) is engageable by an activator component (36) which forms part of a breath-actuated mechanism comprising a vane (38) which is movable in response to inspiration through the mouthpiece (22). The impactor (32) is moved to a primed position during closing of the mouthpiece cover (26) by action of reset arm (40) on activator (36). The impactor (32) is retained in the primed position by the activator component which is held by catch component (37) until the patient inspires through the mouthpiece (22) causing movement of vane (38) which results in the breath-actuation mechanism moving the catch (37) releasing the activator component (36) and the impactor (32). The impactor springs forward such that the cantilevered arm (8) hits the rear of the tape in the middle of the 20mm long impaction region of the tape, thereby releasing powder from the tape into the patient's inspiratory air flow.

The embodiment illustrated in Figure 3 shows an inhaler (102) of the present invention, comprising a housing (104, 104a) defining air inlets (106, 108) and a slit deagglomerator (110) for outlet of the powder aerosol. A mouthpiece (140) clips onto the housing (104, 104a), defining a mouthpiece orifice (142) for insertion into the patient's mouth. Curved surfaces (112, 114) guide the powder aerosol towards slit (110). A desiccant cartridge (116) may be housed in the housing part (104, 104a). A tape supply spool, comprising a flange (not shown) and a hub (118) is mounted in the housing, together with a tape take-up spool, comprising a flange (not shown) and a hub (120). A microdimpled tape (122) coated with micronised drug powder on the side facing the mouthpiece (140) between corners (124, 126) is affixed to the spool hubs (118, 120). The length between corners (124, 126) is the impacted region of the tape and in a preferred embodiment is 12mm long. A mouthpiece part (140) clips removably onto the housing (104a), defining a mouthpiece orifice (142) through which aerosolised drug powder passes to the patient.

The inhaler releases medicament from the impacted region of the tape by striking the rear (uncoated) side of it with the head (shown at (138), coming out of the plane of the figure) of a spring steel impactor (128) which also comprises a two turn coil region (134), loosely positioned around a peg (136) in the housing (104), and a tail (130) bearing on the inside of the housing wall at (132). The impactor is generally similar to that shown in Figure 1 but is cranked as shown in Figure 3, the additional bend in the arm region of the impactor being provided to allow access to the rear of the tape in the shorter (12mm) impaction region of the tape. A release component (144) is pivotally mounted on the housing (104) at pivot pin (152). The release component additionally comprises a flexible arm (146) terminating at a push button (148) at the top of the inhaler, with barbs (150) preventing the button being pulled. Mouthpiece cover (154), pivoted at (136), above the impactor coil (134), comprises a reset arm (156) with surface (158) to push the impactor (128) into a primed position. The impactor (128) in turn pushes on surface (160) of the release component (144), thereby causing it to rotate clockwise and for surface (162) to catch the elbow at the bottom of the impactor head (138) i.e. the intersection of shaft (6) and cantilevered arm (8). In the event of the release component being already in a clockwise position such that it obstructs the impactor during resetting, surface (164) is contacted by the impactor, causing the release component to rotate anti-clockwise until surface (160) is contacted to cause it to rotate clockwise.

Operation of the inhaler is as follows. First, the patient opens the mouthpiece cover (154). In the simplest embodiment of the device the patient then rotates a knob protruding from take-up spool (120), to rotate said spool anti-clockwise by one ratchet or detent position (60°). This is sufficient, with a 23mm diameter hub, to advance 12mm of tape, equivalent to the length between corners (124, 126). After 220 doses of 165µm thick tape, the take-up spool will have grown to around 36mm diameter at a typical tape winding tension. The advanced length for a 60° turn of the knob will then be nearly 19mm, but as the drug dose is determined by the impacted region's length, not by the tape advance length if that is greater, then the drug dose will remain consistent. The supply spool (118) rotates with a fairly high level of friction, in order to ensure the advanced tape remains tight, and non-reverse means (not shown) are provided on the take-up spool (120).

In an alternative embodiment (not shown), an additional arm on the mouthpiece cover (154) pushes on one of a series of radial grooves on an extension protruding from the take-up spool (120), causing it to rotate 60° anti-clockwise upon closing of the mouthpiece cover (154). In a further embodiment (not shown) priming may be effected upon opening the cover.

The patient then places mouthpiece (140) into their mouth, and inhales, causing air to enter the inhaler at inlets (106, 108) and pass the tape to reach the deagglomerator slit (110) and mouthpiece orifice (142). Soon after the start of inhalation the patient pushes button (148), causing the release block (144) to rotate anti-clockwise slightly about pivot pin (152). The causes surface (162) to release the elbow of the impactor head (138), causing the impactor head (138) to travel towards and strike the rear of the impaction region of the tape. This releases a dose of powdered medicament which is entrained in the airstream and passes through the deagglomerator slit (110) which breaks up agglomerates of micronised drug particles to enable them to reach the patient's lungs more effectively.

The patient then closes the mouthpiece cover (154), causing the reset arm (156) to push impactor (128) back until it contacts surface (160) of the release component (144), thereby causing it to rotate clockwise again. This action stores energy in the impactor spring by straining it, tail (130) being unable to move. The impactor (128) is thus primed. The next opening of the cover (154) will then allow the impactor to move forward slightly until the elbow at the bottom of the impactor head (138) is held by surface (162) and the operation cycle thus starts to be repeated.

**Claims**

1. A device for delivering powder comprising a flexible sheet material (27, 122) carrying a powder to be delivered on a surface thereof, structure to support a substantially planar portion of the sheet material and a resettable impactor (32, 128) for dislodging said powder from said portion of the sheet material, the impactor comprising a curved head which is movable between a first, primed position distant from said planar portion of the sheet material, and a second position in which the head impacts a substantially planar portion of said flexible sheet material, the impactor being resiliently biased to propel the head from said first towards said second position, **characterised in that** the impactor (32, 128) comprises:

   (a) a shaft (6) having a first end (2, 130) that is mounted within the device, and
   (b) a cantilevered arm (8, 138) extending from said shaft terminating in a free end, said cantilevered arm comprising the impactor head

      wherein the movement of said shaft between said first and second positions defines a plane of motion,
      at least a portion of said cantilevered arm, disposed intermediate (i) the intersection of said arm with said shaft and (ii) said free end, extending out of a first plane that includes said shaft and is perpendicular to said plane of motion, towards a second plane that is defined by said substantially planar portion of said flexible sheet material,
      a second portion of said cantilevered arm (8, 138) between said intermediate portion and said free end intersects said first plane, defining a curve,
      and said free end extends out of said first plane in a direction away from said second plane.

2. A device as claimed in Claim 1 wherein said curve of the head has a radius of curvature of from about 10mm to about 40mm.

3. A device as claimed in Claim 1 or Claim 2 wherein said shaft and said cantilevered arm are formed from a continuous piece of springing material.

4. A device as claimed in Claim 3 wherein said springy material is spring steel.

5. A device as claimed in Claim 4 wherein the spring steel has a circular cross-section having a diameter of approximately 1mm.

6. A device as claimed in any preceding Claim wherein said head is biased towards said second position by a spring (4).

7. A device as claimed in Claim 6 wherein said spring (4) is positioned adjacent said first end of said shaft (2).

8. A device as claimed in any preceding Claim wherein said cantilevered arm (8) extends at an angle of approximately 90° with respect to said shaft.

9. A device as claimed in any preceding Claim wherein said cantilevered arm (8) has a length of 15mm.

10. A device as claimed in any preceding Claim wherein said sheet material is elongate and said support means comprises a pair of support members (124, 126) disposed in opposed spaced relation along the longitudinal axis of said sheet material.

11. A device as claimed in any preceding Claim wherein said head is biased towards said second position to a sufficient extent so that when said head is released from said primed position it moves towards said second position at a speed of at least 10m/sec.

12. The device as claimed in any preceding Claim further comprising a releasable latch mechanism (37, 162) for retaining said free end in said primed position until use of the device.

13. The device as claimed in Claim 12 further comprising a reset member (40, 156) for returning said free end to said first, primed position after use of the device.

14. The device as claimed in any preceding Claim wherein the powder is micronised.

15. The device as claimed in any preceding Claim wherein the powder is a medicament.

16. The device as claimed in Claim 15 wherein said device is an inhaler.

17. The device as claimed in Claim 16 wherein said device further comprises a housing having a mouthpiece (22, 140) shaped and positioned for delivery of the powder that is dislodged from the sheet to the mouth of a user of the device.

18. The device as claimed in Claim 17 wherein said device further comprises a cover (26, 154) that is movable between a first position, in which it covers said mouthpiece, and a second position in which it is clear of said mouthpiece.

19. The device as claimed in Claim 18 further comprising a releasable latch mechanism for retaining said free end in said primed position until use of the device, and a reset member for returning said free end to said first, primed position after use of the device and said reset member is actuated by movement of said cover between said first and second positions.

20. A device as claimed in any one of Claims 17 to 19 wherein said free end is moveable from said first position to said second position in response to said user inhaling through said mouthpiece, to the device.

**Patentansprüche**

1. , Vorrichtung zum Ausgeben von Pulver, welche ein flexibles Schichtmaterial (27, 122) umfasst, das auf einer Oberfläche desselben ein Pulver trägt, das ausgegeben werden soll, eine Struktur, um einen im Wesentlichen ebenen Abschnitt des Schichtmaterials zu halten, und eine rücksetzbare Aufpralleinrichtung (32, 128) zum Herausklopfen des Pulvers aus dem benannten Abschnitt des Schichtmaterials, wobei die Aufpralleinrichtung einen gebogenen Kopf aufweist, welcher bewegbar ist zwischen einer ersten, funktionsbereiten Stellung in Entfernung von dem ebenen Abschnitt des Schichtmaterials und einer zweiten Stellung, bei welcher der Kopf auf einen im Wesentlichen ebenen Abschnitt des flexiblen Schichtmaterials aufprallt, wobei die Aufpralleinrichtung elastisch vorgespannt ist, um den Kopf aus der ersten in die zweite Stellung vorzutreiben, **dadurch gekennzeichnet,**
**dass** die Aufpralleinrichtung (32, 128) folgendes umfasst:

(a) einen Schaft (6) mit einem ersten Ende (2, 130), welches in der Vorrichtung befestigt ist, und
(b) einen freitragenden Arm (8, 138), der sich von dem Schaft aus erstreckt und in einem freien Ende endet, wobei der freitragende Arm den Aufprallkopf umfasst, wobei

die Bewegung des Schaftes zwischen der ersten und der zweiten Stellung eine Bewegungsebene definiert,

wenigstens ein Abschnitt des freitragenden Armes, der zwischen (i) dem Schnittpunkt des Armes mit dem Schaft und (ii) dem freien Ende vorgesehen ist, sich aus einer ersten Ebene, welche den Schaft beinhaltet, heraus erstreckt und senkrecht zu der Bewegungsebene vorgesehen ist; in Richtung auf eine zweite Ebene, welche durch den im Wesentlichen ebenen Abschnitt des flexiblen Schichtmaterials definiert ist,

ein zweiter Abschnitt des freitragenden Armes (8, 138) zwischen dem mittleren Abschnitt und dem freien Ende die erste Ebene schneidet, wodurch ein Bogen definiert ist,

und sich das freie Ende aus der ersten Ebene heraus in eine Richtung erstreckt, die von der zweiten Ebene weg gerichtet ist.

2. Vorrichtung nach Anspruch 1, wobei der Bogen des Kopfes einen Bogenradius von etwa 10 mm bis etwa 40 mm aufweist.

3. Vorrichtung nach Anspruch 1 oder Anspruch 2, wobei der Schaft und der freitragende Arm aus einem zusammenhängenden Stück federnden Materials ausgebildet sind.

4. Vorrichtung nach Anspruch 3, wobei das federnde Material Federstahl ist.

5. Vorrichtung nach Anspruch 4, wobei der Federstahl einen kreisförmigen Querschnitt mit einem Durchmesser von etwa 1 mm aufweist.

6. Vorrichtung nach einem der vorhergehenden Ansprüche, wobei der Kopf durch eine Feder (4) in Richtung auf die zweite Stellung vorgespannt ist.

7. Vorrichtung nach Anspruch 6, wobei die Feder (4) angrenzend an das erste Ende des Schaftes (2) angeordnet ist.

8. Vorrichtung nach einem der vorhergehenden Ansprüche, wobei sich der freitragende Arm (8) in Bezug auf den Schaft in einem Winkel von etwa 90° erstreckt.

9. Vorrichtung nach einem der vorhergehenden Ansprüche, wobei der freitragende Arm (8) eine Länge von 15 mm aufweist.

10. Vorrichtung nach einem der vorhergehenden Ansprüche, wobei das Schichtmaterial länglich ist und die Halteeinrichtung zwei Halteelemente (124, 126) umfasst, die in gegenüberliegend beabstandeter Beziehung entlang der Längsachse des Schichtmaterials angeordnet sind.

11. Vorrichtung nach einem der vorhergehenden Ansprüche, wobei der Kopf in Richtung auf die zweite Stellung in ausreichendem Maße vorgespannt ist, sodass sich der Kopf, wenn er aus der funktionsbereiten Stellung freigegeben wird, mit einer Geschwindigkeit von wenigstens 10 m/s in Richtung auf die zweite Stellung zu bewegt.

12. Vorrichtung nach einem der vorhergehenden Ansprüche, die ferner einen freigebbaren Varrastungsmechanismus (37, 162) umfasst, um das freie Ende in der funktionsbereiten Stellung zurückzuhalten, bis die Vorrichtung benutzt wird.

13. Vorrichtung nach Anspruch 12, die ferner ein Rückstellelement (40, 156) umfasst, um das freie Ende nach Benutzung der Vorrichtung in die erste, funktionsbereite Stellung zurückzuführen.

14. Vorrichtung nach einem der vorhergehenden Ansprüche, wobei das Pulver mikroskopisch ist.

15. Vorrichtung nach einem der vorhergehenden Ansprüche, wobei das Pulver ein Medikament ist.

16. Vorrichtung nach Anspruch 15, wobei die Vorrichtung ein Inhaliergerät ist.

17. Vorrichtung nach Anspruch 16, wobei die Vorrichtung ferner ein Gehäuse mit einem Mundstück (22, 140) umfasst, das zur Ausgabe des Pulvers geformt und angeordnet ist, welches aus der Schicht zum Mund eines Benutzers der Vorrichtung herausgeklopft wird.

18. Vorrichtung nach Anspruch 17, wobei die Vorrichtung ferner eine Abdeckung (26, 154) umfasst, die zwischen einer ersten Stellung, in welcher sie das Mundstück abdeckt, und einer zweiten Stellung, in welcher das Mundstück freigegeben ist, bewegbar ist.

19. Vorrichtung nach Anspruch 18, die ferner einen freigebbaren Verrastungsmechanismus umfasst, um das freie Ende in der funktionsbereiten Stellung zurückzuhalten, bis die Vorrichtung benutzt wird, und ein Rückstellelement, um das freie Ende nach der Benutzung der Vorrichtung in die erste, funktionsbereite Stellung zurückzuführen, wobei das Rückstellelement durch Bewegung der Abdeckung zwischen der ersten und der zweiten Stellung betätigt wird.

**20.** Vorrichtung nach einem der Ansprüche 17 bis 19, wobei das freie Ende in Reaktion auf das Einatmen des Benutzer durch das Mundstück der Vorrichtung von der ersten Stellung in die zweite Stellung bewegbar ist.

## Revendications

**1.** Appareil de distribution d'une poudre, comprenant un matériau (27, 122) sous forme d'une feuille flexible portant une poudre à distribuer sur une de ses surfaces, une structure de support d'une partie pratiquement plane du matériau en feuille et un organe réarmable de frappe (32, 128) destiné à déloger la poudre de ladite partie du matériau en feuille, l'organe de frappe comportant une tête courbe qui est mobile entre une première position amorcée distante de la partie plane du matériau en feuille, et une seconde position dans laquelle la tête frappe une partie pratiquement plane du matériau en feuille flexible, l'organe de frappe étant rappelé élastiquement afin qu'il propulse la tête de la première position vers la seconde position, **caractérisé en ce que** l'organe de frappe (32, 128) comporte :

(a) un arbre (6) ayant une première extrémité (2, 130) qui est montée dans l'appareil, et
(b) un bras (8, 138) monté en porte-à-faux, s'étendant depuis l'arbre qui se termine à une extrémité libre, le bras en porte-à-faux comprenant la tête d'organe de frappe,

dans lequel le mouvement de l'arbre entre la première et la seconde position délimite un plan de déplacement,
une partie au moins du bras en porte-à-faux, disposée entre (i) l'intersection du bras avec l'arbre et (ii) l'extrémité libre, s'étend vers l'extérieur d'un premier plan qui contient l'arbre et qui est perpendiculaire au plan de déplacement, vers un second plan qui est délimité par la partie sensiblement plane du matériau en feuille flexible,
une seconde partie du bras en porte-à-faux (8, 138) comprise entre la partie intermédiaire et l'extrémité libre recoupa le premier plan en délimitant une courbe, et
l'extrémité libre s'étend vers l'extérieur du premier plan du côté opposé au second plan.

**2.** Appareil selon la revendication 1, dans lequel la courbe de la tête a un rayon de courbure compris entre environ 10 et 40 mm.

**3.** Appareil selon la revendication 1 ou 2, dans lequel l'arbre et le bras en porte-à-faux sont formés d'une pièce continue d'un matériau à ressort.

**4.** Appareil selon la revendication 3, dans lequel le matériau à ressort est un acier à ressort.

**5.** Appareil selon la revendication 4, dans lequel l'acier à ressort a une section circulaire dont le diamètre est d'environ 1 mm.

**6.** Appareil selon l'une quelconque des revendications précédentes, dans lequel la tête est rappelée vers la seconde position par un ressort (4).

**7.** Appareil selon la revendication 6, dans lequel le ressort (4) est positionné près de la première extrémité de l'arbre (2).

**8.** Appareil selon l'une quelconque des revendications précédentes, dans lequel le bras en porte-à-faux (8) s'étend suivant un angle d'environ 90° par rapport à l'arbre.

**9.** Appareil selon l'une quelconque des revendications précédentes, dans lequel le bras en porte-à-faux (8) a une longueur de 15 mm.

**10.** Appareil selon l'une quelconque des revendications précédentes, dans lequel le matériau en feuille est allongé et le dispositif de support comporte une paire d'organes de support (124, 126) disposés à distance et opposés, le long de l'axe longitudinal du matériau en feuille.

**11.** Appareil selon l'une quelconque des revendications précédentes, dans lequel la tête est rappelée vers la seconde position de manière suffisante pour que, lorsque la tête est libérée depuis la position amorcée, elle se déplace vers la seconde position à une vitesse d'au moins 10 m/s.

**12.** Appareil selon l'une quelconque des revendications précédentes, comprenant en outre un mécanisme à verrou amovible (37, 162) destine à retenir l'extrémité libre en position amorcée jusqu'à l'utilisation de l'appareil.

**13.** Appareil selon la revendication 12, comprenant en outre un organe de réarmement (40, 156) destiné à ramener l'extrémité libre vers la première position amorcée après l'utilisation de l'appareil.

**14.** Appareil selon l'une quelconque des revendications précédentes, dans lequel la poudre est micronisée.

**15.** Appareil selon l'une quelconque des revendications précédentes, dans lequel la poudre est un médicament.

**EP 0 634 184 B1**

**16.** Appareil selon la revendication 15, dans lequel l'appareil est un inhalateur.

**17.** Appareil selon la revendication 16, dama lequel l'appareil comporte en outre un boîtier ayant un embout (22, 140) dont la forme et la position permettent la distribution de la poudre qui est délogée de la feuille dans la bouche d'un utilisateur de l'appareil.

**18.** Appareil selon la revendication 17, dans lequel l'appareil comprend en outre un couvercle (26, 154) qui est mobile entre une première position dans laquelle il couvre l'embout, et une seconde position dans laquelle il est dégagé de l'embout.

**19.** Appareil selon la revendication 18, comprenant en outre un mécanisme à verrou amovible destiné à retenir l'extrémité libre en position amorcée jusqu'à l'utilisation de l'appareil, et un organe de réarmement destiné à ramener l'extrémité libre vers la première position amorcée après l'utilisation de l'appareil, et l'organe de réarmement est manoeuvré par le mouvement du couvercle entre la première et la seconde position.

**20.** Appareil selon l'une quelconque des revendications 17 à 19, dans lequel l'extrémité libre est mobile de la première position vers la seconde position lorsque l'utilisateur inhale par l'embout, vers l'appareil.

**Fig. 1a**

**Fig. 1b**

**Fig. 1c**

**Fig. 2**

**Fig. 3**